# FASCICULE DE BREVET EUROPEEN

(11) **EP 1 142 553 B1**
(45) Date de publication et mention de la délivrance du brevet: **07.02.2007**
(21) Numéro de dépôt: 00403474.0
(22) Date de dépôt: 11.12.2000
(51) Int. Cl.: A61Q 5/10, A61K 8/88

(54) **Composition pour la teinture d'oxydation des fibres kératiniques comprenant deux polyammonium quaternaires particuliers**
Zusammensetzungen zur Färbung von keratinischen Fäsern enthaltend zwei spezifische quaternäre Polyammonium
Compositions for dyeing keratinous fibres containing two specific quaternary polyammoniums

(30) Priorité: 30.12.1999 FR 9916764
(43) Date de publication de la demande: 10.10.2001
(73) Titulaire: L'ORÉAL, 75008 Paris (FR)
(72) Inventeur: Bebot, Cecile, 92110 Clichy (FR); Rondeau, Christine, 78500 Sartrouville (FR); Cottard, François, 92300 Levallois Perret (FR); Boudy, Françoise, 75012 Paris (FR)
(74) Mandataire: Miszputen, Laurent

(56) Documents cités:
- EP-A- 0 557 203
- EP-A- 0 673 641
- EP-A- 0 960 617
- DD-B- 213 835
- FR-A- 2 773 992
- GB-A- 2 188 948
- US-A- 4 555 246
- US-A- 4 820 308
- US-A- 4 943 430

## Description

La présente invention concerne une composition pour la teinture d'oxydation des fibres kératiniques, en particulier des fibres kératiniques humaines telles que les cheveux, comprenant, dans un milieu approprié pour la teinture, au moins un colorant d'oxydation, et une association comprenant au moins un cyclohomopolymère de dialkyldiallylammonium et au moins un autre polyammonium quaternaire particulier.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Les agents oxydants permettant la condensation oxydative ont généralement un effet néfaste sur les propriétés des cheveux traités. Les cheveux deviennent rèches, difficilement démêlables et plus fragiles. Pour remédier à ces inconvénients, dans le brevet français 2 270 846, on a déjà proposé d'utiliser certains polyammonium quaternaires.

Cependant, la demanderesse a constaté que les polymères mentionnés ci-dessus ne permettaient pas de remédier suffisamment à ces inconvénients tout en conservant ou en améliorant les propriétés tinctoriales.

Or, après d'importantes recherches menées sur la question, la Demanderesse vient maintenant de découvrir qu'il est possible d'obtenir des compositions de teinture d'oxydation à propriétés cosmétiques améliorées, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) en ajoutant à la composition tinctoriale une association comprenant au moins un cyclohomopolymère de dialkyldiallylammonium et au moins un autre polyammonium quaternaire particulier décrit ci-après.

Ces découvertes sont à la base de la présente invention.

La présente invention a ainsi pour objet une composition pour la teinture d'oxydation des fibres kératiniques, en particulier des fibres kératiniques humaines, telles que les cheveux, comprenant, dans un milieu approprié pour la teinture, au moins un colorant d'oxydation, et caractérisée par le fait qu'elle comprend en outre une association comprenant au moins un cyclopolymère de dialkyldiallylammonium et au moins un autre polyammonium quaternaire particulier décrit ci-après.

Un autre objet de l'invention porte sur une composition prête à l'emploi pour la teinture d'oxydation des fibres kératiniques qui comprend, dans un milieu approprié pour la teinture, au moins un colorant d'oxydation, une association comprenant au moins un cyclohomopolymère de dialkyldiallylammonium et au moins un autre polyammonium quaternaire particulier et au moins un agent oxydant.

Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

L'invention vise également un procédé de teinture d'oxydation des fibres kératiniques, et en particulier des fibres kératiniques humaines telles que les cheveux, consistant à appliquer sur les fibres une composition (A) contenant, dans un milieu approprié pour la teinture, au moins un colorant d'oxydation et une association comprenant au moins un cyclohomopolymère de dialkyldiallylammonium et au moins un autre polyammonium quaternaire particulier décrit ci-après, la couleur étant révélée à pH alcalin, neutre ou acide à l'aide d'une composition (B) contenant au moins un agent oxydant, qui est mélangée juste au moment de l'emploi à la composition (A) ou qui est appliquée séquentiellement sans rinçage intermédiaire.

L'invention vise aussi une variante de ce procédé, qui consiste à appliquer sur les fibres une composition colorante (A) contenant dans un milieu approprié pour la teinture, au moins un colorant d'oxydation, la couleur étant révélée à pH alcalin, neutre ou acide à l'aide d'une composition oxydante (B) qui est mélangée juste au moment de l'emploi à la composition colorante ou qui est appliquée séquentiellement sans rinçage intermédiaire, au moins un cyclohomopolymère de dialkyldiallylammonium et au moins un polyammonium quaternaire particulier étant ensemble présents dans la composition oxydante B, ou séparément dans chacune des compositions A ou B.

L'invention a également pour objet des dispositifs de teinture à plusieurs compartiments ou " kits " pour la teinture d'oxydation des fibres kératiniques, en particulier des fibres kératiniques humaines telles que les cheveux.
De tels dispositifs comportent un premier compartiment contenant au moins un colorant d'oxydation et un deuxième compartiment contenant un agent oxydant, au moins un cyclohomopolymère de dialkyldiallylammonium et au moins un polyammonium quaternaire particulier, étant ensemble présents dans le premier compartiment et/ou dans le second compartiment, ou séparément dans chacun des deux compartiments.

Mais d'autres caractéristiques, aspects, objets et avantages de l'invention apparaîtront encore plus clairement à la lecture de la description et des exemples qui suivent.

Les cyclohomopolymères de dialkyldiallylammonium selon l'invention sont des homopolymères comportant comme constituant principal de la chaîne des motifs de structure (I) suivante : 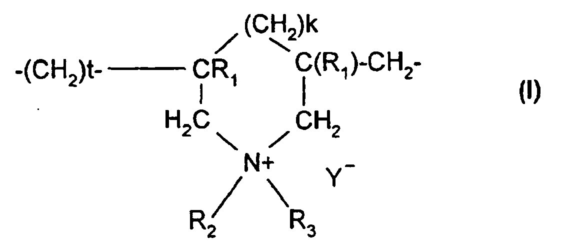 formule (I) dans laquelle k et t sont égaux à 0 ou 1, la somme k + t étant égale à 1 ; R₁ désigne un atome d'hydrogène ou un radical méthyle ; R₂ et R₃, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₂ et R₃ peuvent désigner conjointement avec l'atome d'azote auquel ils sont rattachés, des groupement hétérocycliques, tels que pipéridinyle ou morpholinyle ; R₂ et R₃ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
Parmi les polymères définis ci-dessus, on préfère ceux de formule (I) pour laquelle R₁ désigne l'hydrogène R₂ et R₃ désigne un radical méthyle, et dont le poids moyen est d'environ 100 000.

Parmi ces derniers on peut citer plus particulièrement l'homopolymère de chlorure de diméthyldiallylammonium vendu sous la dénomination "Merquat 100" par la société Calgon (et ses homologues de faible masse moléculaire moyenne en poids).

Les polyammonium quaternaires particuliers selon l'invention sont choisis dans le groupe constitué par :
(i) les polymères constitués de motifs récurrents répondant à la formule (II) suivante : 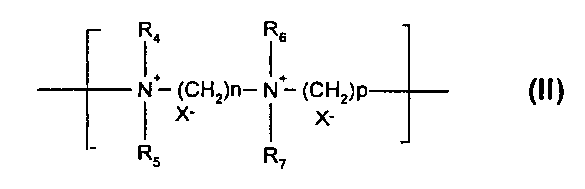 dans laquelle R₄, R₅, R₆ et R₇, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Encore plus particulièrement, on préfère les composés de formule (II) dans laquelle R₄, R₅, R₆ et R₇ représentent un radical méthyle ou éthyle et X⁻ est un atome d'halogène tel qu'un atome de chlore, d'iode ou de brome.
   Des polymères de formule (II) particulièrement préférés sont ceux pour lesquels R₄, R₅, R₆ et R₇, représentent un radical méthyle et n = 3, p = 6 et X = Cl, et notamment ceux dont le poids moléculaire, déterminé par chromatographie par perméation de gel, est compris entre 9500 et 9900 [Polymère W].
   D'autres polymères de formule (II) particulièrement préférés sont ceux pour lesquels R₄ et R₅ représentent un radical méthyle, R₆ et R₇ représentent un radical éthyle et n = p = 3 et X = Br, et notamment ceux dont le poids moléculaire, déterminé par chromatographie par perméation de gel, est d'environ 1200.
   Lesdits polyammonium quaternaires de formule (II) sont préparés et décrits dans le brevet français 2 270 846.
(ii) les polymères constitués de motifs récurrents répondant à la formule (III) suivante :

   -[-N⁺(CH₃)₂-(CH₂)ₚ-NH-CO-D-NH-(CH₂)ₚ-N⁺(CH₃)₂-(CH₂)₂-O-(CH₂)₂-]- 2 X⁻ (III)

   et de préférence de masse moléculaire mesurée par RMN du Carbone 13 inférieure à 100000, formule (III) dans laquelle p désigne un nombre entier variant de 1 à 6 environ, D peut être nul ou peut représenter un groupement -(CH₂)ᵣ-CO- dans lequel r désigne un nombre égal à 4 ou à 7, et X⁻ est un anion dérivé d'un acide minéral
ou organique.

Parmi les polymères de formule (III), on préfère ceux pour lesquels dans la formule (III):
a) D représente un groupement -(CH₂)₄-CO- , X désigne un atome de chlore, la masse moléculaire mesurée par RMN du Carbone 13 ( RMN¹³C) étant d'environ 5600 ; un polymère de ce type est proposé par la société MIRANOL sous le nom de MIRAPOL-AD1,
b) D représente un groupement -(CH₂)₇-CO-, X désigne un atome de chlore, la masse moléculaire mesurée par RMN du Carbone 13 ( RMN¹³C) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).
   Plus particulièrement encore, on préfère selon l'invention le polymère de formule (III) dans laquelle D désigne la valeur zéro, X désigne un atome de chlore, la masse moléculaire mesurée par RMN du Carbone 13, ( RMN¹³C) étant d'environ 25500.

Lesdits polyammonium quaternaires de formule (III) peuvent être préparés selon les procédés décrits dans les brevets U.S.A. n° 4 157 388, 4 390 689, 4 702 906, 4 719 282.

Dans la composition selon l'invention, la quantité pondérale du cyclohomopolymère de dialkyldiallylammonium est préférentiellement comprise entre environ 0,05 et 5%, et encore plus préférentiellement entre 0,1 et 3%, et celle du polyammonium quaternaire à motifs de formule (II) ou (III) varie de préférence entre environ 0,05 et 10%, et encore plus préférentiellement entre 0,2 et 5%, par rapport au poids total de la composition. Dans la composition, le rapport pondéral du polyammonium quaternaire à motifs de formule (II) ou (III) au cyclohomopolymère de dialkyldiallylammonium de formule (I) est compris entre 0,1 et 50 environ et de préférence entre 1 et 10 environ.

### Colorants d'oxydation

Les colorants d'oxydation utilisables selon l'invention sont choisis parmi les bases d'oxydation et/ou les coupleurs.
De préférence les compositions selon l'invention contiennent au moins une base d'oxydation.
Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- **(I)** les paraphénylènediamines de formule (I) suivante et leurs sels d'addition avec un acide : 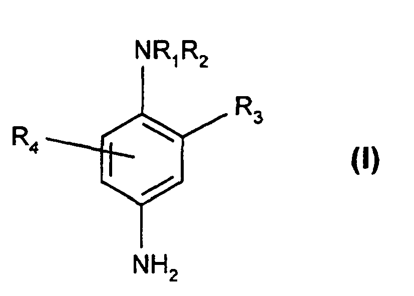 dans laquelle :
   R₁ représente un atome d'hydrogène, un radical alkyle en C₁-C₄, monohydroxyalkyle en C₁-C₄, polyhydroxyalkyle en C₂-C₄ , alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.
      Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
      Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
      Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 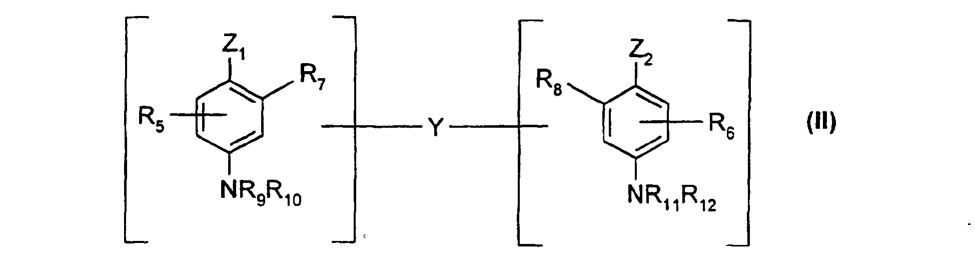 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un
   ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
      étant entendu que les composés de formule (II) ne comportent qu'un seul bras de liaison Y par molécule.

   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-bis-(éthyl)-N, N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- **(III)** les para-aminophénols répondant à la formule (III) suivante, et leurs sels d'addition avec un acide : 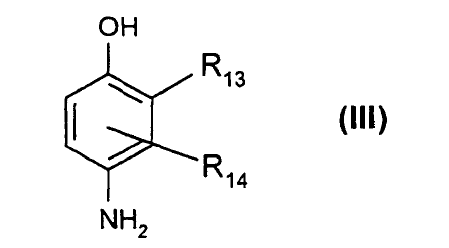 dans laquelle :
   **R₁₃** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R₁₄** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine la pyrazolo-[1,5-a]-pyrimidine-3,5-diamine ; la2,7-diméthyl-pyrazolo-[1,5-a]-pyrimidine-3,5-diamine; le 3-amino-pyrazolo-[1,5-a]-pyrimidin-7-ol ; le 3-amino-pyrazolo-[1,5-a]-pyrimidin-5-ol; le 2-(3-amino pyrazolo-[1,5-a]-pyrimid in-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition prête à l'emploi selon l'invention comprend de préférence dans la composition colorante (A) et/ou dans la composition oxydante (B) en outre au moins un polymère épaississant de type non ionique, anionique, ou cationique comportant au moins une chaîne grasse.

### Polymères épaississants comportant au moins une chaîne grasse

Parmi les polymères épaississants comportant au moins une chaîne grasse et de type anionique, on peut citer :
- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = CR'CH₂OBₙR (I)
dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
Des polymères amphiphiles anioniques de ce type sont décrits et préparés, selon un procédé de polymérisation en émulsion, dans le brevet EP-0 216 479.
Parmi ces polymères épaississants anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
- **(II)** ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 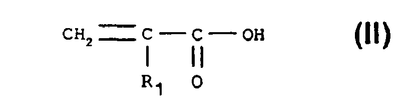 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 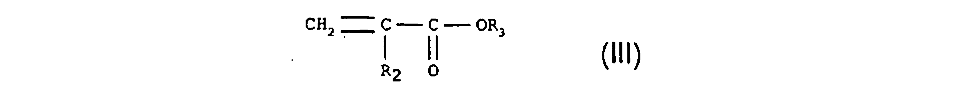 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères épaississants anioniques à chaîne grasse, on utilisera plus particulièrement des polymères formés à partir d'un mélange de monomères comprenant :
   (i) essentiellement de l'acide acrylique,
   (ii) un ester de formule (III) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
      Parmi ce type de polymères épaississants anioniques à chaîne grasse, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment.
      Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .
- **(III)** les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
- **(IV)** les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique/acrylate de méthyle/diméthyl métaisopropényl benzyl isocyanate d'alcool béhényle éthoxylé (40OE) en dispersion aqueuse à 25%.
- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.
   Préférentiellement ces composés comprennent également comme monomère un ester d'acide carboxylique à insaturation α,β-monoéthylénique et d'alcool en C1C4.
   A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères épaississants à chaîne grasse de type non ionique, utilisés selon l'invention, sont choisis de préférence parmi :
- **(1)** les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- **(2)** les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- **(3)** les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- **(7)** les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂₋₁₄ et le produit ELFACOS T212 à chaîne alkyle en C₁₈ de chez AKZO.
Le produit DW 1206B de chez ROHM & HAAS à chaîne alkyle en C₂₀ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères épaississants à chaîne grasse de type cationique utilisés selon la présente invention sont choisis de préférence parmi les dérivés de cellulose quaternisée et les polyacrylates à groupements latéraux aminés non cycliques.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
   On peut indiquer comme exemples d'alkylhydroxyéthyl-celluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).
Comme exemples de polyacrylates à chaînes latérales aminées, on peut citer les polymères 8781- 121B ou 9492-103 proposés par la société NATIONAL STARCH.

Dans la composition de teinture d'oxydation selon l'invention, on préfère utiliser un polymère épaississant à chaîne grasse de type non ionique.

Les polymères épaississants à chaîne grasse de type anionique, non ionique ou cationique sont utilisés de préférence en une quantité pouvant varier d'environ 0,01 à 10% en poids du poids total de la composition de teinture. Plus préférentiellement, cette quantité varie d'environ 0,1 à 5% en poids.

La composition prête à l'emploi selon l'invention comprend également de préférence dans la composition colorante (A) et/ou dans la composition oxydante (B) un ou plusieurs tensioactifs.

### Tensio-actifs

Le ou les tensioactifs peuvent être indifféremment choisis, seuls ou en mélanges, au sein des tensioactifs anioniques, amphotères, non ioniques, zwittérioniques et cationiques.
Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, et les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention tout comme les alcools gras éthoxylés.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₃₄ -CONHCH₂CH₂ -N(R₃₅)(R₃₆)(CH₂COO⁻)

dans laquelle : R₃₄ désigne un radical alkyle d'un acide R₃₄-COOH présent dans l'huile de coprah hydrolysée, un radical heptyle, nonyle ou undécyle, R₃₅ désigne un groupement bêta-hydroxyéthyle et R₃₆ un groupement carboxyméthyle ;
et

R₃₄'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₃₄' désigne un radical alkyle d'un acide R₃₇ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,1 à 30% du poids total de la composition.

La composition prête à l'emploi selon l'invention peut également contenir dans la composition colorante (A) et/ou la composition oxydante (B) d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.
Ces épaississants d'appoint peuvent représenter de 0,01 à 10% en poids du poids total de la composition.

Le milieu de la composition approprié pour la teinture est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras peuvent représenter de 0,001 à 20% en poids environ du poids total de la composition.

Bien entendu, l'homme de l'art veillera à choisir le ou les éventuels composés complémentaires mentionnés ci-avant, de manière telle que les propriétés avantageuses attachées intrinsèquement à la composition prête à l'emploi selon l'invention ne soient pas, ou substantiellement pas, altérées par la ou les adjonctions envisagées.

Dans la composition oxydante (B), l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition colorante (A) ou de la composition prête à l'emploi et appliquée sur les fibres kératiniques [composition résultant du mélange de la composition tinctoriale (A) et de la composition oxydante (B)], est généralement compris entre les valeurs 4 et 12. II est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (IV) suivante : 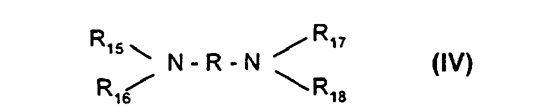 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Des exemples concrets illustrant l'invention sont indiqués ci-après, sans pour autant présenter un caractère limitatif.

### EXEMPLES

On a préparé les compositions suivantes :

**Composition oxydante :**

| | |
|---|---|
| Alcool gras | 2,3 g |
| Alcool gras oxyéthyléné | 0,6 g |
| Amide grasse | 0,9 g |
| Glycérine | 0,5 g |
| Peroxyde d'hydrogène | 7,5 g |
| parfum | qs |
| Eau déminéralisée qsp | 100 g |

**Composition colorante:**

| | |
|---|---|
| (exprimée en grammes) | |
| Mélange d'alcools linéaires en C18 à C24 [C18/C20/C22/C24, 7/58/30/6, teneur en alcools>95%] (NAFOL 20-22) | 3 |
| Mélange d'alcools linéaires en C18 à C24 [C18/C20/C22/C24, 7/58/30/6, teneur en alcools>95%] oxyéthylénés 30 OE (NAFOLOX 20-22 300E) | 1,35 |
| Alcool stéarylique oxyéthyléné 2 OE | 4 |
| Alcool stéarylique oxyéthyléné 21 OE | 2 |
| Acide oléique | 2,6 |
| Distéarate de glycol | 2 |
| Propylène glycol | 5 |
| Monoisopropanolamide d'acides de coprah | 2 |
| Aculyn 44 vendu par la société ROHM & HAAS | 1,4 MA* |
| Acide polyacrylique réticulé | 0,6 |
| Polyammonium quaternaire (VV) | 3 MA* |
| Merquat 100 vendu par la société CALGON | 0,4 MA* |
| Réducteurs | 0,7 |
| Séquestrants | 0,2 |
| 1,3-dihydroxybenzène (résorcinol) | 0,6 |
| 1,4-diaminobenzène | 0,5 |
| 1-hydroxy-3-amino-benzène | 0,1 |
| 1-hydroxy-2-amino-benzène | 0,05 |
| 1-hydroxy-4-amino-benzène | 0,09 |
| 6-hydroxy-benzomorpholine | 0,017 |
| 1-β-hydroxyéthyloxy-2,4-diamino-benzène, dichlorhydrate | 0,039 |
| Monobutyléther de propylène glycol | 2,5 |
| Monoéthanolamine pure | 1,06 |
| Ammoniaque (à 20,5% en ammoniac) | 11,1 |
| Eau qsp | 100 |

| | |
|---|---|
| MA*= Matière Active | |

La composition colorante a été mélangée, au moment de l'emploi, dans un bol en plastique et pendant 2 minutes, à la composition oxydante donnée ci-dessus, à raison de 1 partie de composition colorante pour 1,5 partie de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing, à nouveau rincées à l'eau, puis séchées et démélées.
Les cheveux ont alors été teints dans une nuance chatain clair puissante.

Des résultats du même type ont été obtenus en remplaçant dans l'exemple ci-dessus, le polyammonium quaternaire (W) par la même quantité de Miranol A15 vendu par la société MIRANOL.

## Revendications

1. Composition pour la teinture d'oxydation des fibres kératiniques, en particulier des fibres kératiniques humaines telles que les cheveux, comprenant, dans un milieu approprié pour la teinture, au moins un colorant d'oxydation, **caractérisée par le fait qu'**elle contient en outre une association comprenant au moins un cyclohomopolymère de dialkyldiallylammonium comportant comme constituant de la chaîne des motifs de formule (I) suivante : 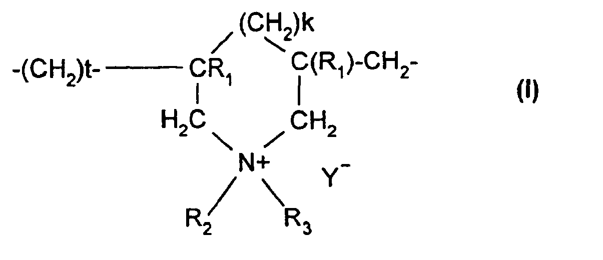 dans laquelle k et t sont égaux à 0 ou 1, la somme k + t étant égale à 1 ; R₁ désigne un atome d'hydrogène ou un radical méthyle ; R₂ et R₃, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₂ et R₃ peuvent désigner conjointement avec l'atome d'azote auquel ils sont rattachés, des groupement hétérocycliques, tels que pipéridinyle ou morpholinyle ; R₂ et R₃ indépendamment l'un de l'autre désignent de préférence un groupement alkyle ayant de 1 à 4 atomes de carbone ; Y⁻ est un anion dérivé d'un acide minéral ou organique ;
et au moins un polyammonium quaternaire constitué de motifs récurrents de formule (II) ou (III) suivantes : 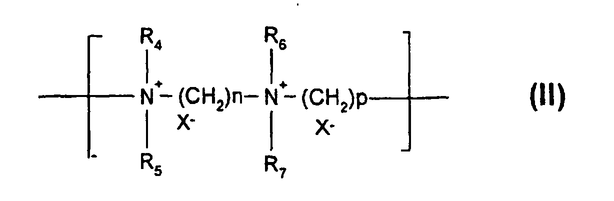 formule (II) dans laquelle R₄, R₅, R₆ et R₇, identiques ou différents, désignent un radical alkyle ou hydroxyalkyle ayant de 1 à 4 atomes de carbone environ, n et p sont des nombres entiers variant de 2 à 20 environ et X- est un anion dérivé d'un acide minéral ou organique ;
-[-N⁺(CH₃)₂-(CH₂)ₚ-NH-CO-D-NH-(CH₂)ₚ-N⁺(CH₃)₂-(CH₂)₂-O-(CH₂)₂-]- 2X⁻ (III)
formule (III) dans laquelle p désigne un nombre entier variant de 1 à 6 environ, D est nul ou représente un groupement -(CH₂)ᵣ-CO- dans lequel r désigne un nombre égal à 4 ou à 7 et X⁻ est un anion dérivé d'un acide minéral ou organique.

2. Composition selon la revendication 1, **caractérisée par le fait que** dans la formule (I), R₁ désigne l'hydrogène, R₂ et R₃ désignent méthyle et Y- désigne le chlore.

3. Composition selon la revendication 1, **caractérisée par le fait que** dans la formule (II), R₄, R₅, R₆ et R₇ désignent méthyle ou éthyle et X- est un atome d'halogène.

4. Composition selon la revendication 3, **caractérisée par le fait que** dans la formule (II), R₄, R₅, R₆ et R₇ désignent méthyle, n = 3, p = 6 et X- désigne le chlore.

5. Composition selon la revendication 3, **caractérisée par le fait que** dans la formule (II), R₄, R₅, désignent méthyle, R₆ et R₇ désignent éthyle, n = p = 3 et X- désigne le brome.

6. Composition selon la revendication 1, **caractérisée par le fait que** dans la formule (III), D est nul, X désigne le chlore.

7. Composition selon l'une quelconque des revendications 1 ou 2, **caractérisée par le fait que** le cyclohomopolymère de dialkyldiallylammonium à motifs de formule (I) représente en poids de 0,05 à 5%, de préférence de 0,1 à 3% du poids total de la composition.

8. Composition selon l'une quelconque des revendications 1 ou 3 à 6, **caractérisée par le fait que** le polyammonium quaternaire à motifs de formule (II) ou (III) représente en poids de 0,05 à 10%, de préférence de 0,2 à 5% du poids total de la composition.

9. Composition selon l'une quelconque des revendications précédentes, **caractérisée par le fait que** le rapport pondéral du polyammonium quaternaire à motifs de formule (II) ou (III) au cyclohomopolymère de dialkyldiallylammonium de formule (I) est compris entre 0,1 et 50.

10. Composition selon la revendication 9, **caractérisée par le fait que** le rapport pondéral du polyammonium quaternaire à motifs de formule (II) ou (III) au cyclohomopolymère de dialkyldiallylammonium de formule (I) est compris entre 1 et 10.

11. Composition selon l'une quelconque des revendications précédentes, **caractérisée par le fait que** le colorant d'oxydation est choisi parmi les bases d'oxydation et les coupleurs.

12. Composition selon l'une quelconque des revendications précédentes, **caractérisée par le fait qu'**elle contient au moins une base d'oxydation.

13. Composition selon les revendications 11 ou 12, **caractérisée par le fait que** les bases d'oxydation sont choisis parmi les ortho- ou para- phénylènediamines, les bases doubles, les ortho- ou para- aminophénols, et les bases hétérocycliques, ainsi que les sels d'addition de ces composés avec un acide.

14. Composition selon la revendication 13 **caractérisée par le fait que** les paraphénylènediamines sont choisies parmi les composés de structure (I) suivante : 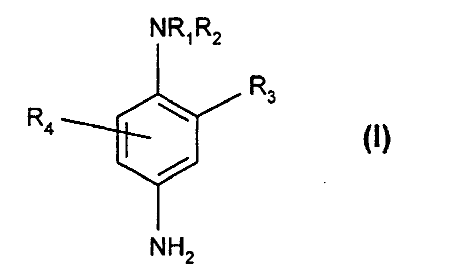 dans laquelle:
R₁ représente un atome d'hydrogène, un radical alkyle en C₁-C₄, monohydroxyalkyle en C₁-C₄, polyhydroxyalkyle en C₂-C₄ , alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

15. Composition selon la revendication 13, **caractérisée par le fait que** les bases doubles sont choisies parmi les composés de structure (II) suivante : 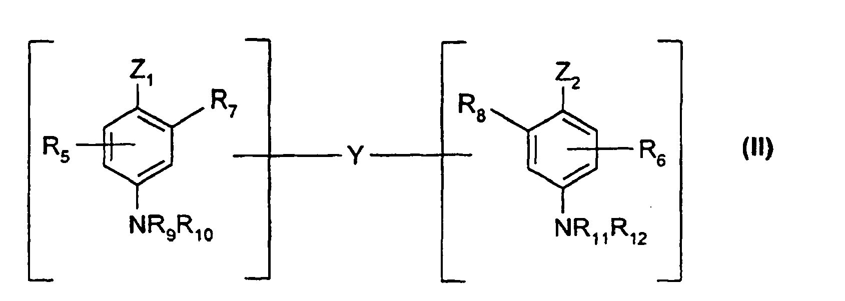 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

16. Composition selon les revendications 14 ou 15, **caractérisée par le fait que** les groupements azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

17. Composition selon la revendication 13, **caractérisée par le fait que** les para-aminophénols sont choisis parmi les composés de structure (III) suivante : 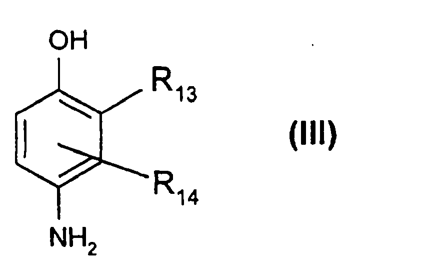 dans laquelle :
**R₁₃** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
**R₁₄** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

18. Composition selon la revendication 13, **caractérisée par le fait que** les bases hétérocycliques sont choisies parmi les dérivés pyridiniques, les dérivés pyrimidiniques dont les pyrazolopyrimidines, et les dérivés pyrazoliques.

19. Composition selon les revendications 11 à 18, **caractérisée par le fait que** les bases d'oxydation sont présentes dans des concentrations allant de 0,0005 à 12% en poids par rapport au poids total de la composition.

20. Composition selon les revendications 11 ou 12 , **caractérisée par le fait que** les coupleurs sont choisis parmi les métaphénylènediamines, les métaaminophénols, les métadiphénols, les coupleurs hétérocycliques, et les sels d'addition de ces composés avec un acide.

21. Composition selon les revendications 11, 12 ou 20, **caractérisée par le fait que** les coupleurs sont présents dans des concentrations allant de 0,0001 à 10% en poids par rapport au poids total de la composition.

22. Composition selon les revendications 13 ou 20, **caractérisée par le fait que** les sels d'addition avec un acide des bases d'oxydation et des coupleurs sont choisis parmi les chlorhydrates, les bromhydrates, les sulfates, les tartrates, les lactates et les acétates.

23. Composition selon l'une quelconque des revendications précédentes, **caractérisée par le fait qu'**elle contient en outre des colorants directs.

24. Composition selon l'une quelconque des revendications précédentes, **caractérisée par le fait qu'**elle contient en outre au moins un agent réducteur, dans des quantités allant de 0,05 à 3% en poids par rapport au poids total de la composition.

25. Composition selon l'une quelconque des revendications précédentes, **caractérisée par le fait qu'**elle contient en outre un ou plusieurs alcools gras.

26. Composition selon la revendication 25, **caractérisée par le fait que** le ou les alcools gras représentent en poids de 0,001 à 20% du poids total de la composition.

27. Composition prête à l'emploi pour la teinture d'oxydation des fibres kératiniques en particulier des fibres kératiniques humaines telles que les cheveux **caractérisée par le fait qu'**elle est obtenue par mélange d'une composition (A) telle que définie à l'une quelconque des revendications 1 à 26 et d'une composition (B) contenant au moins un agent oxydant.

28. Composition selon la revendication 27, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

29. Composition selon la revendication 28, **caractérisée par le fait que** l'agent oxydant est le peroxyde d'hydrogène.

30. Composition selon la revendication 29, **caractérisée par le fait que** l'agent oxydant est une solution d'eau oxygénée dont le titre varie de 1 à 40 volumes.

31. Composition selon l'une quelconque des revendications 1 à 30, **caractérisée par le fait qu'**elle possède un pH allant de 4 à 12.

32. Composition selon la revendication 27, **caractérisée par le fait que** la composition (A) et/ou la composition (B) contient au moins un polymère épaississant comportant au moins une chaîne grasse.

33. Composition selon la revendication 32, **caractérisée par le fait que** le polymère épaississant à chaîne grasse est de type non-ionique.

34. Composition selon l'une quelconque des revendications 32 ou 33, **caractérisée par le fait que** le ou les polymères épaississants à chaîne grasse sont présents à raison de 0,01 à 10% et de préférence de 0,1 à 5% en poids du poids total de la composition.

35. Composition selon la revendication 27, **caractérisée par le fait que** la composition (A) et/ou la composition (B) contient au moins un tensioactif choisi parmi les tensioactifs anioniques, cationiques, non ioniques ou amphotères.

36. Composition selon la revendication 35, **caractérisée par le fait que** les tensioactifs représentent 0,01 à 40% et de préférence 0,1 à 30% du poids total de la composition.

37. Composition selon la revendication 27, **caractérisée par le fait que** la composition (A) et/ou la composition (B) contient un agent épaississant choisi parmi les dérivés de cellulose, les dérivés de guar, les gommes d'origine microbienne, les épaississants synthétiques ne possédant pas une chaîne grasse.

38. Composition selon la revendication 37, **caractérisée par le fait que** le ou les agents épaississants représentent de 0,01 à 10% en poids du poids total de la composition.

39. Procédé de teinture des fibres kératiniques et en particulier des fibres kératiniques humaines telles que les cheveux, **caractérisé par le fait qu'**il consiste à appliquer sur les fibres une composition de teinture (A) contenant, dans un milieu approprié pour la teinture, au moins un colorant d'oxydation et une association comprenant au moins un cyclohomopolymère de dialkyldiallylammonium à motifs de formule (I) telle que définie aux revendications 1 ou 2 et au moins un polyammonium quaternaire à motifs de formule (II) ou (III) telle que définie à l'une quelconque des revendications 1 et 3 à 6, la couleur étant révélée à pH alcalin, neutre ou acide à l'aide d'une composition (B) contenant au moins un agent oxydant, qui est mélangée juste au moment de l'emploi à la composition (A) ou qui est appliquée séquentiellement sans rinçage intermédiaire.

40. Procédé de teinture des fibres kératiniques et en particulier des fibres kératiniques humaines telles que les cheveux, **caractérisé par le fait qu'**il consiste à appliquer sur les fibres une composition colorante (A) contenant dans un milieu approprié pour la teinture, au moins un colorant d'oxydation, la couleur étant révélée à pH alcalin, neutre ou acide à l'aide d'une composition oxydante (B) qui est mélangée juste au moment de l'emploi à la composition colorante ou qui est appliquée séquentiellement sans rinçage intermédiaire, au moins un cyclohomopolymère de dialkyldiallylammonium à motifs de formule (I) telle que définie aux revendications 1 ou 2 et au moins un polyammonium quaternaire à motifs de formule (II) ou (III) telle que définie à l'une quelconque des revendications 1 et 3 à 6, étant ensemble présents dans la composition oxydante B, ou séparément dans chacune des compositions A ou B.

41. Dispositif à plusieurs compartiments ou " Kit" pour la teinture d'oxydation des fibres kératiniques et en particulier des fibres kératiniques humaines telles que les cheveux, **caractérisé par le fait qu'**il comporte au moins deux compartiments, dont l'un d'entre eux contient au moins un colorant d'oxydation et l'autre compartiment contient un agent oxydant, au moins un cyclohomopolymère de dialkyldiallylammonium à motifs de formule (I) telle que définie aux revendications 1 ou 2 et au moins un polyammonium quaternaire à motifs de formule (II) ou (III) telle que définie à l'une quelconque des revendications 1 et 3 à 6, étant ensemble présents dans le premier compartiment et/ou dans le second compartiment, ou séparément dans chacun des deux compartiments.

## Claims

1. Composition for the oxidation dyeing of keratinous fibres, in particular human keratinous fibres such as hair, comprising, in an appropriate dyeing medium, at least one oxidation dye, **characterized in that** it comprises, in addition, a combination comprising at least one cyclohomopolymer of dialkyldiallylammonium comprising, as constituent of the chain, units having the following formula (I) : 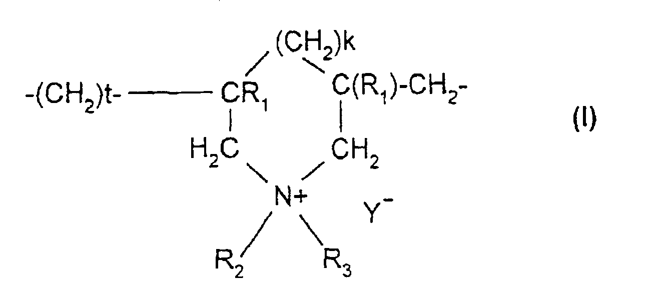 in which k and t are equal to 0 or 1, the sum k + t being equal to 1; R₁ denotes a hydrogen atom or a methyl radical; R₂ and R₃, independently of each other, denote an alkyl group having from 1 to 22 carbon atoms, a hydroxyalkyl group in which the alkyl group preferably has 1 to 5 carbon atoms, a lower (C₁-C₄) amidoalkyl group, or R₂ and R₃ may denote, jointly with the nitrogen atom to which they are attached, heterocyclic groups such as piperidinyl or morpholinyl; R₂ and R₃, independently of each other, preferably denote an alkyl group having from 1 to 4 carbon atoms; Y⁻ is an anion derived from an inorganic or organic acid;
and at least one quaternary polyammonium consisting of repeat units having the following formula (II) or (III): 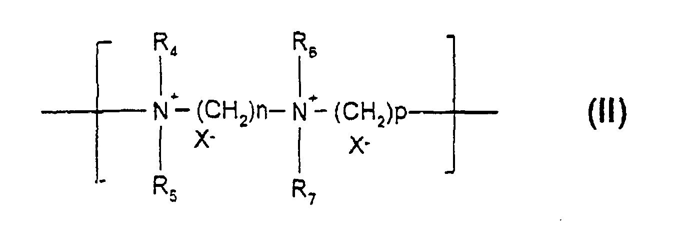 in which formula (II) R₄, R₅, R₆ and R₇, which are identical or different, denote an alkyl or hydroxyalkyl radical having from 1 to 4 carbon atoms approximately, n and p are integers varying from 2 to 20 approximately and X⁻ is an anion derived from an inorganic or organic acid;
-[-N⁻ (CH₃)₂-(CH₂)ₚ-NH-CO-D-NH-(CH₂)ₚ-(CH₃)₂-(CH₂)₂-O-(CH₂)₂-]- 2X⁻ (III)
in which formula (III) p denotes an integer ranging from 1 to 6 approximately, D is zero or represents a group -(CH₂)ᵣ-CO- in which r denotes a number equal to 4 or to 7, and X⁻ is an anion derived from an inorganic or organic acid.

2. Composition according to Claim 1, **characterized in that** in formula (I), R₁ denotes hydrogen, R₂ and R₃ denote methyl and Y⁻ denotes chlorine.

3. Composition according to Claim 1, **characterized in that** in formula (II), R₄, R₅, R₆ and R₇ denote methyl or ethyl and X⁻ is a halogen atom.

4. Composition according to Claim 3, **characterized in that** in formula (II), R₄, R₅, R₆ and R₇ denote methyl, n = 3, p = 6 and X⁻ denotes chlorine.

5. Composition according to Claim 3, **characterized in that** in formula (II), R₄, R₅ denote methyl, R₆ and R₇ denote ethyl, n = p = 3 and X⁻ denotes bromine.

6. Composition according to Claim 1, **characterized in that** in formula (III), D is zero, X denotes chlorine.

7. Composition according to either one of Claims 1 and 2, **characterized in that** the cyclohomopolymer of dialkyldiallylammonium with units of formula (I) represents, by weight, from 0.05 to 5%, preferably from 0.1 to 3%, of the total weight of the composition.

8. Composition according to any one of Claims 1 or 3 to 6, **characterized in that** the quaternary polyammonium with units of formula (II) or (III) represents, by weight, from 0.05 to 10%, preferably from 0.2 to 5%, of the total weight of the composition.

9. Composition according to any one of the preceding claims, **characterized in that** the weight ratio of the quaternary polyammonium with units of formula (II) or (III) to the cyclohomopolymer of dialkyldiallylammonium of formula (I) is between 0.1 and 50.

10. Composition according to Claim 9, **characterized in that** the weight ratio of the quaternary polyammonium with units of formula (II) or (III) to the cyclohomopolymer of dialkyldiallylammonium of formula (I) is between 1 and 10.

11. Composition according to any one of the preceding claims, **characterized in that** the oxidation dye is chosen from oxidation bases and couplers.

12. Composition according to any one of the preceding claims, **characterized in that** it contains at least one oxidation base.

13. Composition according to Claim 11 or 12, **characterized in that** the oxidation bases are chosen from ortho- or para-phenylenediamines, double bases, ortho- or para-aminophenols and heterocyclic bases, as well as the addition salts of these compounds with an acid.

14. Composition according to Claim 13, **characterized in that** the para-phenylenediamines are chosen from the compounds having the following structure (I) : 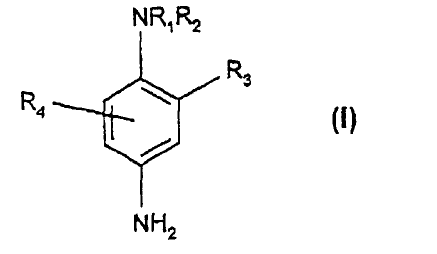 in which:
R₁ represents a hydrogen atom, a C₁-C₄ alkyl radical, a monohydroxy(C₁-C₄ alkyl) radical, a polyhydroxy(C₂-C₄ alkyl) radical, a (C₁-C₄) alkoxy (C₁-C₄) alkyl radical, a C₁-C₄ alkyl radical substituted with a nitrogen-containing group, a phenyl radical or a 4'-aminophenyl radical;
R₂ represents a hydrogen atom, a C₁-C₄ alkyl radical, a monohydroxy(C₁-C₄ alkyl) radical or a polyhydroxy(C₂-C₄ alkyl) radical, a (C₁-C₄)alkoxy(C₁-C₄)alkyl radical or a C₁-C₄ alkyl radical substituted with a nitrogen-containing group;
R₁ and R₂ may also form with the nitrogen atom carrying them a 5- or 6-membered nitrogen-containing heterocycle optionally substituted with one or more alkyl, hydroxyl or ureido groups;
R₃ represents a hydrogen atom, a halogen atom such as a chlorine atom, a C₁-C₄ alkyl radical, a sulpho radical, a carboxyl radical, a monohydroxy(C₁-C₄ alkyl) radical or a hydroxy(C₁-C₄ alkoxy) radical, an acetylamino(C₁-C₄ alkoxy) radical, a mesylamino(C₁-C₄ alkoxy) radical or a carbamoylamino(C₁-C₄ alkoxy) radical,
R₄ represents a hydrogen or halogen atom or a C₁-C₄ alkyl radical.

15. Composition according to Claim 13, **characterized in that** the double bases are chosen from the compounds having the following structure (II): 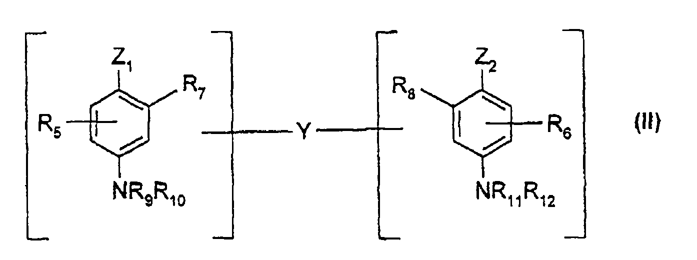 in which:
- Z₁ and Z₂, which are identical or different, represent a hydroxyl or -NH₂ radical which may be substituted with a C₁-C₄ alkyl radical or with a linking arm Y;
- the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a monohydroxy(C₁-C₄ alkyl) radical, a polyhydroxy(C₂-C₄ alkyl) radical, an amino(C₁-C₄ alkyl) radical or a linking arm Y;
- R₇, R₈, R₉, R₁₀, R₁₁ and R₁₂, which are identical or different, represent a hydrogen atom, a linking arm Y or a C₁-C₄ alkyl radical; it being understood that the compounds of formula (II) contain only one linking arm Y per molecule.

16. Composition according to Claim 14 or 15, **characterized in that** the nitrogen-containing groups are chosen from amino, mono (C₁-C₄) alkylamino, (C₁-C₄) dialkylamino, (C₁-C₄) trialkylamino, monohydroxy (C₁-C₄) alkylamino, imidazolinium and ammonium radicals.

17. Composition according to Claim 13, **characterized in that** the para-aminophenols are chosen from the compounds having the following structure (III): 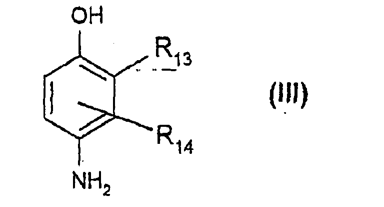 in which:
R₁₃ represents a hydrogen atom, a halogen atom such as fluorine, a C₁-C₄ alkyl, monohydroxy(C₁-C₄ alkyl), (C₁-C₄) alkoxy(C₁-C₄) alkyl or amino (C₁-C₄ alkyl) or hydroxy (C₁-C₄) alkylamino (C₁-C₄ alkyl) radical,
R₁₄ represents a hydrogen atom or a halogen atom such as fluorine, a C₁-C₄ alkyl, monohydroxy(C₁-C₄ alkyl), polyhydroxy(C₂-C₄ alkyl), amino(C₁-C₄ alkyl), cyano(C₁-C₄ alkyl) or (C₁-C₄) alkoxy(C₁-C₄)alkyl radical.

18. Composition according to Claim 13, **characterized in that** the heterocyclic bases are chosen from pyridine derivatives, pyrimidine derivatives including pyrazolopyrimidines, and pyrazole derivatives.

19. Composition according to Claims 11 to 18, **characterized in that** the oxidation bases are present in concentrations ranging from 0.0005 to 12% by weight relative to the total weight of the composition.

20. Composition according to Claim 11 or 12, **characterized in that** the couplers are chosen from meta-phenylenediamines, meta-aminophenols, meta-diphenols, heterocyclic couplers, and the addition salts of these compounds with an acid.

21. Composition according to Claim 11, 12 or 20, **characterized in that** the couplers are present in concentrations ranging from 0.0001 to 10% by weight relative to the total weight of the composition.

22. Composition according to Claim 13 or 20, **characterized in that** the addition salts with an acid of the oxidation bases and of the couplers are chosen from hydrochlorides, hydrobromides, sulphates, tartrates, lactates and acetates.

23. Composition according to any one of the preceding claims, **characterized in that** it contains, in addition, direct dyes.

24. Composition according to any one of the preceding claims, **characterized in that** it contains, in addition, at least one reducing agent, in quantities ranging from 0.05 to 3% by weight relative to the total weight of the composition.

25. Composition according to any one of the preceding claims, **characterized in that** it contains, in addition, one or more fatty alcohols.

26. Composition according to Claim 25, **characterized in that** the fatty alcohol(s) represent by weight from 0.001 to 20% of the total weight of the composition.

27. Ready-to-use composition for the oxidation dyeing of keratinous fibres, in particular human keratinous fibres such as hair, **characterized in that** it is obtained by mixing a composition (A) as defined in any one of Claims 1 to 26 and a composition (B) containing at least one oxidizing agent.

28. Composition according to Claim 27, **characterized in that** the oxidizing agent is chosen from hydrogen peroxide, urea peroxide, alkali metal bromates or ferricyanides, persalts, oxidation-reduction enzymes such as laccases, peroxidases and oxidoreductases containing 2 electrons, where appropriate in the presence of their respective donor or cofactor.

29. Composition according to Claim 28, **characterized in that** the oxidizing agent is hydrogen peroxide.

30. Composition according to Claim 29, **characterized in that** the oxidizing agent is a solution of hydrogen peroxide whose titre varies from 1 to 40 volumes.

31. Composition according to any one of Claims 1 to 30, **characterized in that** it possesses a pH ranging from 4 to 12.

32. Composition according to Claim 27, **characterized in that** the composition (A) and/or the composition (B) contains at least one thickening polymer comprising at least one fatty chain.

33. Composition according to Claim 32, **characterized in that** the thickening polymer containing a fatty chain is of nonionic type.

34. Composition according to either one of Claims 32 and 33, **characterized in that** the thickening polymer(s) containing a fatty chain are present in an amount of from 0.01% to 10% and preferably from 0.1% to 5% by weight of the total weight of the composition.

35. Composition according to Claim 27, **characterized in that** the composition (A) and/or the composition (B) contains at least one surfactant chosen from anionic, cationic, nonionic or amphoteric surfactants.

36. Composition according to Claim 35, **characterized in that** the surfactants represent 0.01 to 40% and preferably 0.1 to 30% of the total weight of the composition.

37. Composition according to Claim 27, **characterized in that** the composition (A) and/or the composition (B) contains a thickening agent chosen from cellulose derivatives, guar derivatives, gums of microbial origin, synthetic thickeners which do not possess a fatty chain.

38. Composition according to Claim 37, **characterized in that** the thickening agent(s) represent from 0.01 to 10% by weight of the total weight of the composition.

39. Method of dyeing keratinous fibres and in particular human keratinous fibres such as hair, **characterized in that** it consists in applying to the fibres a dyeing composition (A) containing, in an appropriate dyeing medium, at least one oxidation dye and a combination comprising at least one cyclohomopolymer of dialkyldiallylammonium with units of formula (I) as defined in Claim 1 or 2 and at least one quaternary polyammonium with units of formula (II) or (III) as defined in any one of Claims 1 and 3 to 6, the colour being developed at alkaline, neutral or acidic pH with the aid of a composition (B) containing at least one oxidizing agent, which is mixed just at the time of use with the composition (A) or which is applied sequentially without intermediate rinsing.

40. Method of dyeing keratinous fibres and in particular human keratinous fibres such as hair, **characterized in that** it consists in applying to the fibres a dyeing composition (A) containing, in an appropriate dyeing medium, at least one oxidation dye, the colour being developed at alkaline, neutral or acidic pH with the aid of an oxidizing composition (B) which is mixed just at the time of use with the dyeing composition or which is applied sequentially without intermediate rinsing, at least one cyclohomopolymer of dialkyldiallylammonium with units of formula (I) as defined in Claim 1 or 2 and at least one quaternary polyammonium with units of formula (II) or (III) as defined in any one of Claims 1 and 3 to 6, being present together in the oxidizing composition B, or separately in each of the compositions A or B.

41. Multicompartment device or "kit" for the oxidation dyeing of keratinous fibres and in particular human keratinous fibres such as hair, **characterized in that** it comprises at least two compartments, one of which contains at least one oxidation dye and the other compartment contains an oxidizing agent, at least one cyclohomopolymer of dialkyldiallylammonium with units of formula (I) as defined in Claim 1 or 2 and at least one quaternary polyammonium with units of formula (II) or (III) as defined in any one of Claims 1 and 3 to 6, being present together in the first compartment and/or in the second compartment, or separately in each of the two compartments.

## Patentansprüche

1. Zusammensetzung zum oxidativen Färben von Keratinfasern, insbesondere menschlichen Keratinfasern, wie Haaren, die in einem zum Färben geeigneten Medium mindestens einen Oxidationsfarbstoff enthält, **dadurch gekennzeichnet, dass** sie ferner eine Kombination enthält, die mindestens ein Dialkyldiallylammonium-Cyclohomopolymer mit Wiederholungseinheiten der folgenden Formel (I): 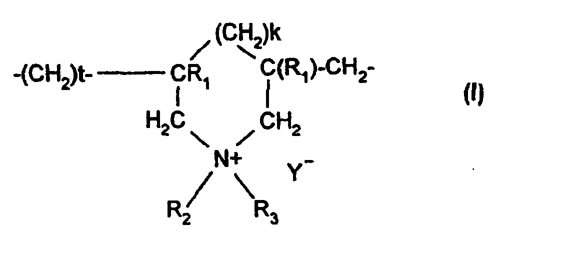 wobei in der Formel (I) k und t 0 oder 1 sind, wobei die Summe k + t 1 bedeutet; R₁ ein Wasserstoffatom oder Methyl bedeutet; R₂ und R₃ unabhängig voneinander eine Alkylgruppe mit 1 bis 22 Kohlenstoffatomen, eine Hydroxyalkylgruppe, bei der die Alkylgruppe vorzugsweise 1 bis 5 Kohlenstoffatome aufweist, eine niedere (C₁₋₄) Amidoalkylgruppe bedeuten oder R₂ und R₃ gemeinsam mit dem Stickstoffatom, an das sie gebunden sind, heterocyclische Gruppen bilden können, wie Piperidinyl oder Morpholinyl; wobei R₂ und R₃ unabhängig voneinander vorzugsweise eine Alkylgruppe mit 1 bis 4 Kohlenstoffatomen bedeuten; und Y⁻ ein Anion ist, das von einer anorganischen oder organischen Säure abgeleitet ist; und mindestens eine quartäre Polyammoniumverbindung mit Wiederholungseinheiten der folgenden Formeln (II) oder (III) umfasst: 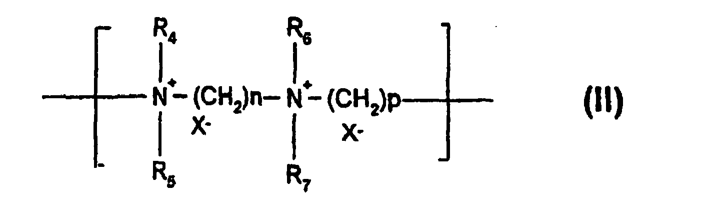 wobei in der Formel (II) die Gruppen R₄, R₅, R₆ und R₇, die gleich oder verschieden sind, eine Alkyl- oder Hydroxyalkylgruppe mit etwa 1 bis 4 Kohlenstoffatome bedeuten, n und p ganze Zahlen im Bereich von etwa 2 bis 20 sind und X- ein von einer anorganischen oder organischen Säure abgeleitetes Anion bedeutet;
-{-N⁺(CH₃)₂-(CH₂)ₚ-NH-CO-D-NH-(CH₂)ₚ-N⁺(CH₃)₂-(CH₂)₂-O-(CH₂)₂-]- 2X (III)
wobei in der Formel (III) p eine ganze von etwa 1 bis 6 bedeutet, D nicht vorhanden sein kann oder eine Gruppe -(CH₂)ᵣ-CO- bedeuten kann, wobei r 4 oder 7 bedeutet, und X⁻ ein Anion ist, das von einer anorganischen oder organischen Säure abgeleitet ist.

2. Zusammensetzung nach Anspruch 1, **dadurch gekennzeichnet, dass** in der Formel (I) R₁ Wasserstoff, R₂ und R₃ Methyl und Y-Chlor bedeutet.

3. Zusammensetzung nach Anspruch 1, **dadurch gekennzeichnet, dass** in der Formel (II) R₄, R₅, R₆ und R₇ Methyl oder Ethyl und X- ein Halogenatom bedeutet.

4. Zusammensetzung nach Anspruch 3, **dadurch gekennzeichnet, dass** in der Formel (II) R₄, R₅, R₆ und R₇ Methyl bedeutet, n = 3, p = 6 und X⁻ Chlor bedeutet.

5. Zusammensetzung nach Anspruch 3, **dadurch gekennzeichnet, dass** in der Formel (II) R₄ und R₅ Methyl bedeutet, R₆ und R₇ Ethyl bedeutet, n = p = 3 und X- Brom bedeutet.

6. Zusammensetzung nach Anspruch 1, **dadurch gekennzeichnet, dass** in der Formel (III) D nicht vorhanden ist und X Chlor bedeutet.

7. Zusammensetzung nach einem der Ansprüche 1 oder 2, **dadurch gekennzeichnet, dass** das Dialkyldiallylammonium-Cyclohomopolymer mit Einheiten der Formel (I) 0,05 bis 5 Gew.-% und vorzugsweise 0,1 bis 3 % des Gesamtgewichts der Zusammensetzung ausmacht.

8. Zusammensetzung nach einem der Ansprüche 1 oder 3 bis 6, **dadurch gekennzeichnet, dass** die quartäre Polyammoniumverbindung mit Einheiten der Formel (II) oder (III) 0,05 bis 10 Gew.-% und vorzugsweise 0,2 bis 5 % des Gesamtgewichts der Zusammensetzung ausmacht.

9. Zusammensetzung nach einem der vorhergehenden Ansprüche, **dadurch gekennzeichnet, dass** das Gewichtsverhältnis der quartären Polyammoniumverbindung mit Einheiten der Formel (II) oder (III) und des Dialkyldiallylammonium-Cyclohomopolymers der Formel (I) im Bereich von 0,1 bis 50 liegt.

10. Zusammensetzung nach Anspruch 9, **dadurch gekennzeichnet, dass** das Gewichtsverhältnis der quartären Polyammoniumverbindung mit Einheiten der Formel (II) oder (III) und des Dialkyldiallylammonium-Cyclohomopolymers der Formel (I) im Bereich von 1 bis 10 liegt.

11. Zusammensetzung nach einem der vorhergehenden Ansprüche, **dadurch gekennzeichnet, dass** der Oxidationsfarbstoff unter den Oxidationsbasen und Kupplern ausgewählt ist.

12. Zusammensetzung nach einem der vorhergehenden Ansprüche, **dadurch gekennzeichnet, dass** die Zusammensetzung mindestens eine Oxidationsbase enthält.

13. Zusammensetzung nach den Ansprüchen 11 oder 12, **dadurch gekennzeichnet, dass** die Oxidationsbasen unter den *o*- oder p-Phenylendiaminen, Doppelbasen, *o*- oder *p*-Aminophenolen und den heterocyclischen Basen sowie den Additionssalzen dieser Verbindungen mit einer Säure ausgewählt sind.

14. Zusammensetzung nach Anspruch 13, **dadurch gekennzeichnet, dass** die *p*-Phenylendiamine unter den Verbindungen der folgenden Formel (I) ausgewählt sind: 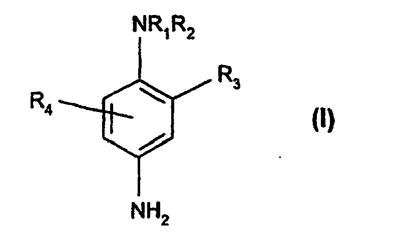 worin bedeuten:
- R₁ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer Stickstoff-haltigen Gruppe substituierte C₁₋₄-Alkylgruppe, Phenyl oder 4'-Aminophenyl;
- R₂ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄) oder eine mit einer Stickstoff-haltigen Gruppe substituierte C₁₋₄-Alkylgruppe,
- wobei R₁ und R₂ auch mit dem Stickstoffatom, von dem sie getragen werden, einen 5- oder 6-gliedrigen Stickstoffheterocyclus bilden können, der gegebenenfalls mit einer oder mehreren Gruppen Alkyl, Hydroxy oder Ureido substituiert ist;
- R₃ ein Wasserstoffatom, ein Halogenatom, wie Chlor, C₁₋₄-Alkyl, Sulfo, Carboxy, C₁₋₄-Monohydroxyalkyl, C₁₋₄-Hydroxy-alkoxy, C₁₋₄-Acetylaminoalkoxy, C₁₋₄-Mesylaminoalkoxy oder C₁₋₄-Carbamoylaminoalkoxy,
- R₄ ein Wasserstoffatom, ein Halogenatom oder C₁₋₄-Alkyl.

15. Zusammensetzung nach Anspruch 13, **dadurch gekennzeichnet, dass** die Doppelbasen unter den Verbindungen der folgenden Struktur (II) ausgewählt sind: 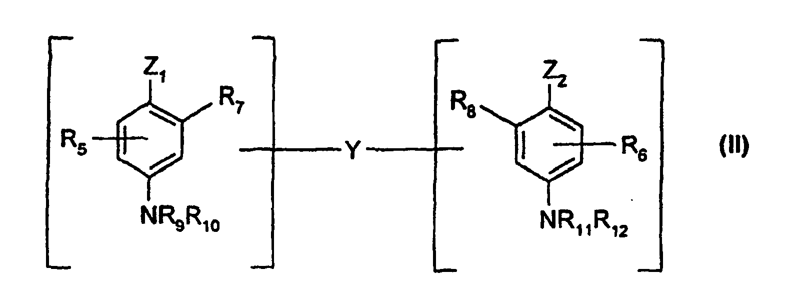 worin bedeuten:
- die Gruppen Z₁ und Z₂, die gleich oder verschieden sind, die Gruppe Hydroxy oder -NH₂, die mit einer C₁₋₄-Alkylgruppe oder einer Verbindungsgruppe Y substituiert sein kann;
- die Verbindungsgruppe Y eine geradkettige oder verzweigte Alkylenkette mit 1 bis 14 Kohlenstoffatomen, die durch eine oder mehrere Stickstoff-haltige Gruppen und/oder ein oder mehrere Heteroatome, wie Sauerstoff, Schwefel oder Stickstoff, unterbrochen oder abgeschlossen sein kann und gegebenenfalls mit einer oder mehreren Hydroxygruppen oder C₁-₆-Alkoxygruppen substituiert ist;
- die Gruppen R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- die Gruppen R₇, R₈, R₉, R₁₀, R₁₁ und R₁₂, die gleich oder verschieden sind, ein Wasserstoffatom, eine Verbindungsgruppe Y oder eine C₁₋₄-Alkylgruppe;
- mit der Maßgabe, dass die Verbindungen der Formel (II) nur eine Verbindungsgruppe Y pro Molekül enthalten.

16. Zusammensetzung nach den Ansprüchen 14 oder 15, **dadurch gekennzeichnet, dass** die Stickstoff-haltigen Gruppen unter den Gruppen Amino, Monoalkyl(C₁₋₄)amino, Dialkyl(C₁₋₄)amino, Trialkyl(C₁₋₄)amino, Monohydroxyalkyl(C₁₋₄)amino, Imidazolinium und Ammonium ausgewählt sind.

17. Zusammensetzung nach Anspruch 13, **dadurch gekennzeichnet, dass** die *p*-Aminophenole unter den Verbindungen der folgenden Struktur (III) ausgewählt sind: 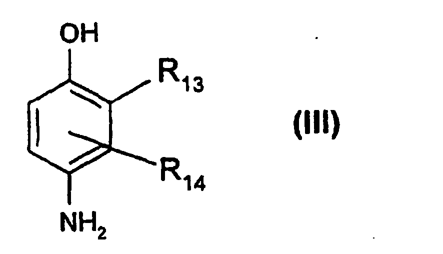 worin bedeuten:
- R₁₃ ein Wasserstoffatom, ein Halogenatom, wie Fluor, C₁₋₄-Alkyl, C₁₋₄-Monohydroxyalkyl, Alkoxy(C₁₋₄)alkyl(C₁₋₄), C₁₋₄-Aminoalkyl oder Hydroxyalkyl(C₁₋₄)aminoalkyl(C₁₋₄),
- R₁₄ ein Wasserstoffatom, ein Halogenatom, wie Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder Alkoxy(C₁₋₄)alkyl(C₁₋₄).

18. Zusammensetzung nach Anspruch 13, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

19. Zusammensetzung nach den Ansprüchen 11 bis 18, **dadurch gekennzeichnet, dass** die Oxidationsbasen in Konzentrationen von 0,0005 bis 12 Gew.-%, bezogen auf das Gesamtgewicht der Zusammensetzung, enthalten sind.

20. Zusammensetzung nach Anspruch 11 oder 12, **dadurch gekennzeichnet, dass** die Kuppler unter den *m*-Phenylendiaminen, *m*-Aminophenolen, *m*-Dihydroxybenzolen, heterocyclischen Kupplern und den Additionssalzen dieser Verbindungen mit einer Säure ausgewählt sind.

21. Zusammensetzung nach den Ansprüchen 11,12 oder 20, **dadurch gekennzeichnet, dass** die Kuppler in Konzentrationen von 0,0001 bis 10 Gew.-%, bezogen auf das Gesamtgewicht der Zusammensetzung, enthalten sind.

22. Zusammensetzung nach den Ansprüchen 13 oder 20, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

23. Zusammensetzung nach einem der vorhergehenden Ansprüche, **dadurch gekennzeichnet, dass** sie ferner Direktfarbstoffe enthält.

24. Zusammensetzung nach einem der vorhergehenden Ansprüche, **dadurch gekennzeichnet, dass** sie ferner mindestens ein Reduktionsmittel in Mengenanteilen von 0,05 bis 3 Gew.-%, bezogen auf das Gesamtgewicht der Zusammensetzung enthält.

25. Zusammensetzung nach einem der vorhergehenden Ansprüche, **dadurch gekennzeichnet, dass** sie ferner einen oder mehrere Fettalkohole enthält.

26. Zusammensetzung nach Anspruch 25, **dadurch gekennzeichnet, dass** der oder die Fettalkohole 0,001 bis 20 % des Gesamtgewichts der Zusammensetzung ausmachen.

27. Gebrauchsfertige Zusammensetzung zum oxidativen Färben von Keratinfasern, insbesondere menschlichen Keratinfasern, wie Haaren, **dadurch gekennzeichnet, dass** sie durch Mischen einer in den Ansprüchen 1 bis 26 definierten Zusammensetzung (A) und einer Zusammensetzung (B), die mindestens ein Oxidationsmittel enthält, erhalten wird.

28. Zusammensetzung nach Anspruch 27, **dadurch gekennzeichnet, dass** das Oxidationsmittel unter Wasserstoffperoxid, Harnstoffperoxid, Alkalimetallbromaten oder Alkalimetallferricyaniden, Salzen von Persäuren, Redoxenzymen, wie Laccasen, Peroxidasen und Oxidoreductasen (2 Elektronen) gegebenenfalls in Gegenwart ihres jeweiligen Donors oder Cofaktors ausgewählt ist.

29. Zusammensetzung nach Anspruch 28, **dadurch gekennzeichnet, dass** es sich bei dem Oxidationsmittel um Wasserstoffperoxid handelt.

30. Zusammensetzung nach Anspruch 29, **dadurch gekennzeichnet, dass** das Oxidationsmittel eine Wasserstoffperoxidlösung ist, deren Titer im Bereich von 1 bis 40 Volumina liegt.

31. Zusammensetzung nach einem der Ansprüche 1 bis 30, **dadurch gekennzeichnet, dass** sie einen pH-Wert von 4 bis 12 aufweist.

32. Zusammensetzung nach Anspruch 27, **dadurch gekennzeichnet, dass** die Zusammensetzung (A) und/ oder die Zusammensetzung (B) mindestens verdickendes Polymer mit mindestens einer Fettkette enthält.

33. Zusammensetzung nach Anspruch 32, **dadurch gekennzeichnet, dass** das verdickende Polymer mit Fettkette vom nichtionischen Typ ist

34. Zusammensetzung nach einem der Ansprüche 32 oder 33, **dadurch gekennzeichnet, dass** das oder die verdickenden Polymere mit Fettkette 0,01 bis 10 Gew.-% und vorzugsweise 0,1 bis 5 Gew.-% des Gesamtgewichts der Zusammensetzung ausmachen.

35. Zusammensetzung nach Anspruch 27, **dadurch gekennzeichnet, dass** die Zusammensetzung (A) und/oder die Zusammensetzung (B) mindestens einen grenzflächenaktiven Stoff enthält, der unter den anionischen, kationischen, nichtionischen oder amphoteren grenzflächenaktiven Stoffen ausgewählt ist.

36. Zusammensetzung nach Anspruch 35, **dadurch gekennzeichnet, dass** die grenzflächenaktiven Stoffe 0,01 bis 40 % und vorzugsweise 0,1 bis 30 % des Gesamtgewichts der Zusammensetzung, ausmachen.

37. Zusammensetzung nach Anspruch 27, **dadurch gekennzeichnet, dass** die Zusammensetzung (A) und/oder die Zusammensetzung (B) ein Verdickungsmittel enthält, das unter den Cellulosederivaten, Guarderivaten, Gummen mikrobieller Herkunft, synthetischen Verdickungsmitteln, die keine Fettkette aufweisen, ausgewählt ist.

38. Zusammensetzung nach Anspruch 37, **dadurch gekennzeichnet, dass** das oder die Verdickungsmittel 0,01 bis 10 Gew.-% des Gesamtgewichts der Zusammensetzung ausmachen.

39. Verfahren zum Färben von Keratinfasern und insbesondere menschlichen Keratinfasern, wie Haaren, **dadurch gekennzeichnet, dass** es darin besteht, auf die Fasern eine Farbmittelzusammensetzung (A) aufzutragen, die in einem zum Färben geeigneten Medium mindestens einen Oxidationsfarbstoff und eine Kombination enthält, die mindestens ein Dialkyldiallylammonium-Cyclohomopolymer mit Einheiten der Formel (I), wie es in den Ansprüchen 1 oder 2 definiert wurde, und mindestens eine quartäre Polyammoniumverbindung mit Einheiten der Formel (II) oder (III), wie sie in den Ansprüchen 1 und 3 bis 6 definiert wurde, umfasst, und die Farbe bei einem alkalischen, neutralen oder sauren pH-Wert mit einer Zusammensetzung (B) zu bilden, die mindestens ein Oxidationsmittel enthält und bei der Anwendung mit der Zusammensetzung (A) vermischt wird oder ohne zwischenzeitliches Spülen getrennt davon anschließend aufgetragen wird.

40. Verfahren zum Färben von Keratinfasern und insbesondere menschlichen Keratinfasern, wie Haaren, **dadurch gekennzeichnet, dass** es darin besteht, auf die Fasern eine Farbmittelzusammensetzung (A) aufzutragen, die in einem zum Färben geeigneten Medium mindestens einen Oxidationsfarbstoff enthält, und die Farbe bei einem alkalischen, neutralen oder sauren pH-Wert mit Hilfe einer oxidierenden Zusammensetzung (B) zu bilden, die bei der Anwendung mit der Farbmittelzusammensetzung vermischt wird oder ohne zwischenzeitliches Spülen getrennt davon anschließend aufgetragen wird, wobei mindestens ein Dialkyldiallylammonium-Cyclohomopolymer mit Einheiten der Formel (I), wie es in den Ansprüchen 1 oder 2 definiert wurde, und mindestens eine quartäre Polyammoniumverbindung mit Einheiten der Formel (II) oder (III), wie sie in den Ansprüchen 1 und 3 bis 6 definiert wurde, gemeinsam in der oxidierenden Zusammensetzung (B) enthalten sind oder getrennt voneinander in jeder der Zusammensetzungen (A) oder (B).

41. Vorrichtung mit mehreren Abteilungen oder "Kit" zum oxidativen Färben von Keratinfasern und insbesondere menschlichen Keratinfasern, wie Haaren, **dadurch gekennzeichnet, dass** mindestens zwei Abteilungen enthalten sind, wobei eine Abteilung mindestens eine Oxidationsbase und eine andere Abteilung ein Oxidationsmittel enthält, wobei mindestens ein Dialkyldiallylammonium-Cyclohomopolymer mit Einheiten der Formel (I), wie es in den Ansprüchen 1 oder 2 definiert wurde, und mindestens eine quartäre Polyammoniumverbindung mit Einheiten der Formel (II) oder (III), wie sie in den Ansprüchen 1 und 3 bis 6 definiert wurde, gemeinsam in der ersten Abteilung und/oder in der zweiten Abteilung enthalten sind oder getrennt voneinander in jeder der beiden Zusammensetzungen.
